# EUROPEAN PATENT SPECIFICATION

(11) **EP 2 865 759 B1**
(45) Date of publication and mention of the grant of the patent: **17.05.2017**
(21) Application number: 13806525.5
(22) Date of filing: 19.06.2013
(51) Int. Cl.: C12N 15/113, A61K 31/711, C07H 21/04

(54) **NUCLEIC ACID COMPLEX AND NUCLEIC ACID-POLYSACCHARIDE COMPLEX**
NUKLEINSÄUREKOMPLEX UND NUKLEINSÄURE-POLYSACCHARID-KOMPLEX
COMPLEXE D'ACIDES NUCLÉIQUES ET COMPLEXE ACIDE NUCLÉIQUE-POLYSACCHARIDE

(30) Priority: 20.06.2012 JP 2012139250
(43) Date of publication of application: 29.04.2015
(73) Proprietor: NapaJen Pharma, Inc., Burlingame, CA 94010 (US)
(72) Inventor: SAKURAI Kazuo, Himeji-shi Hyogo 670-0033 (JP); MOCHIZUKI Shin-ichi, Fukuoka-shi Fukuoka 812-0063 (JP); TANAKA Motoko, Kitakyushu-shi Fukuoka 808-0136 (JP); HIGUCHI Sadaharu, Koganei-shi Tokyo 184-8588 (JP)
(74) Representative: Cabinet Plasseraud
(86) International application number: PCT/JP2013/066887
(87) International publication number: WO 2013/191223

(56) References cited:
- EP-A1- 2 226 384
- WO-A1-2007/058323
- WO-A1-2009/078470
- WO-A1-2012/020795
- JP-A- 2005 204 612
- JP-A- 2009 512 673
- TAKEDATSU H. ET AL.: "The New Therapeutic Approach for Inflammatory Bowel Disease UsingAntisense Macrophage-Migration Inhibitory Factor (MIF) / Schizophyllan(SPG) Complex", GASTROENTEROLOGY, vol. 140, no. 5, 1 May 2011 (2011-05-01), pages S520-S520, XP002741159,
- SHINICHI MOCHIZUKI ET AL: "ssDNA-dsRNAs are cleaved at the next to its chimera-junction point by an unknown RNase activity", BIOCHEMICAL AND BIOPHYSICAL RESEARCH COMMUNICATIONS, vol. 428, no. 4, 1 November 2012 (2012-11-01), pages 433-437, XP055197016, ISSN: 0006-291X, DOI: 10.1016/j.bbrc.2012.10.100
- MOCHIZUKI SHINICHI ET AL: "Macrophage specific delivery of TNF-[alpha] siRNA complexed with [beta]-1,3-glucan inhibits LPS-induced cytokine production in a murine acute hepatitis model", BIOORGANIC & MEDICINAL CHEMISTRY, PERGAMON, GB, vol. 21, no. 9, 5 March 2013 (2013-03-05), pages 2535-2542, XP028545187, ISSN: 0968-0896, DOI: 10.1016/J.BMC.2013.02.035
- SHINICHI MOCHIZUKI ET AL: "Dectin-1 targeting delivery of TNF-[alpha] antisense ODNs complexed with [beta]-1,3-glucan protects mice from LPS-induced hepatitis", JOURNAL OF CONTROLLED RELEASE, vol. 151, no. 2, 1 April 2011 (2011-04-01) , pages 155-161, XP055197019, ISSN: 0168-3659, DOI: 10.1016/j.jconrel.2011.01.026
- SAKURAI K ET AL: "Polysaccharide-polynucleotide complexes. 2. Complementary polynucleotide mimic behavior of the natural polysaccharide schizophyllan in the macromolecular complex with single-stranded RNA and DNA", BIOMACROMOLECULES, AMERICAN CHEMICAL SOCIETY, US, vol. 2, no. 3, 1 October 2001 (2001-10-01) , pages 641-650, XP002504242, ISSN: 1525-7797, DOI: 10.1021/BM000121R [retrieved on 2001-06-12]
- INOUE, T: "Oligonucleotide therapeutics: past, present and future", DRUG DELIVERY SYSTEM, vol. 31, no. 1, 2016, pages 15-23,
- KIMURA, T. ET AL.: "Polysaccharide-Polynucleotide Complexes (III): A novel interaction between the beta-1,3-Glucan family and the single stranded RNA Poly(C)", CHEMISTRY LETTERS 2000, 1 August 2000 (2000-08-01), pages 1242-1243,

## Description

### Technical Field

The present disclosure relates to a technology to enhance the serum stability of a nucleic acid conjugate comprising DNA and RNA.

### Background Art

The reading of the human genome was completed in the year 2003, which is the 50th year since the discovery of the DNA duplex structure in the year 1953. Currently, action mechanisms and interactions of a variety of proteins are being investigated. Furthermore, it has been found recently that non-protein-coding RNAs regulate transcription and translation of genes. A technology to regulate biological functions by using bioactive short artificial nucleic acids (nucleic acid medicine) is proposed as one method applying such research results.

Small interfering RNAs (siRNAs) are low-molecular-weight double-stranded RNAs of 21-23 base pairs. siRNAs are involved in a phenomenon called RNA interference (RNAi) (see Non Patent Literature 1), in which messenger RNAs (mRNA) are inhibited in a sequence-specific manner and thereby the expression of the encoded proteins is suppressed. This phenomenon is believed to have evolved as a part of host defense mechanisms against virus infection and the like. The design of siRNAs is feasible from sequence information alone and, therefore, a large-scale screening as seen in the development of conventional small molecule drugs is not needed and, furthermore, siRNAs are believed to have very few side effects because of the ability thereof to specifically inhibit particular genes, so that siRNA is expected to be a class of next generation therapeutics.

However, RNA, which is a naturally occurring phosphate ester-type nucleic acid, loses the activity thereof in a quite short time period due to nucleases and non-specific adsorption of proteins *in vivo.* Because of this problem, medicine derived from naturally occurring nucleic acids has not provided any significant effect in clinical research with human. To solve the problem associated with naturally occurring nucleic acids that lose the activities thereof in a short time period in biological environments or in culture media, a number of chemically modified nucleic acids, in which naturally occurring nucleic acids are chemically modified, have been proposed. For example, nucleic acids, in which the hydroxyl group of the 2' position of a ribose is chemically modified by a methoxy group (2'-O-methyl) (see Non Patent Literature 2) or a fluorine (F) (see Non Patent Literature 3), or chemically modified to form a locked nucleic acid (LNA) (see Non Patent Literature 4), are particularly known. Moreover, it is reported that such chemical modifications may improve the binding affinity of an siRNA to a target mRNA.

Such chemically modified nucleic acids are called nucleic acid analogues. Nucleic acid analogues have succeeded in extending greatly the time to lose the activity as compared with naturally occurring nucleic acids. This is because nucleases cannot recognize the nucleic acid analogues. However, the toxicity of nucleic acid analogues, such as non-specific adsorption of proteins *in vivo,* unexpected bioactivities, development of severe hepatic disorder and the like, all of which are attributed to the unnatural origins of the nucleic acid analogues, has become a problem.

A technology for introducing nucleic acid into cells has also been proposed, in which a naturally occurring nucleic acid is included in a biocompatible compound that improves the membrane permeability as well as protects the nucleic acid from degradation. Retroviruses (see Non Patent Literature 5) or adenoviruses (see Non Patent Literature 6) or the like provided a very promising result as gene carriers *in vitro* but risks associated with these naturally derived viruses, such as inflammatory and immunogenic properties, and spontaneous mutagenesis and integration into a cellular genome, are pointed out and the use of these viruses *in vivo* is restricted.

Thus, the use of a non-viral carrier composed of artificial materials as an alternative of the naturally derived gene carriers has been presented, which non-viral carrier is not only easier to handle but also capable of concentrating DNA on cells in a more reliable and more efficient manner than the virus-based carriers (see Non Patent Literature 7). A polycationic polymer modified with polyethylene glycol (see Non Patent Literature 8), polyethyleneimine (see Non Patent Literature 9), block copolymers of a cationic polymer (see Non Patent Literature 10), dendrimers (see Non Patent Literature 11) and the like have been so far developed as non-viral artificial carriers for nucleic acid. However, the safety of such cationic polymers has not been confirmed. The presence of amino groups is essential for polymers to be cationic but the amino group is highly bioactive, which may cause a risk of toxicity *in vivo,* and the like.

The inventors have so far paid attention to β-1,3-glucan as a class of gene carrier and found that β-1,3-glucans form a new type of complex with a nucleic acid medicine (an antisense-strand DNA, a CpG DNA) (see Patent Literatures 1 and 2, Non Patent Literatures 12, 13, and 14).

The inventors have found that a triple helical complex comprising one molecule of nucleic acid and two molecules of polysaccharide is formed by dissolving a β-1,3-glucan, which exists naturally in the form of a triple helix, in an aprotonic polar organic solvent such as dimethylsulfoxide (DMSO) or an alkaline solution having a concentration of not less than 0.1 N to dissociate the triple helix and to form single-stranded molecules, followed by addition of single-stranded nucleic acid and exchange of the solvent with water or bringing of the pH of the solution to neutral. In this case, the polysaccharide molecules and the nucleic acid molecule in the triple helical complex are considered to form the intermolecular bonding through hydrogen bonding and hydrophobic interaction (see Non Patent Literature 15).

As described above, the nucleic acid to be complexed with β-1,3-glucan is a single-stranded nucleic acid and, in particular, polydeoxyadenine (poly dA) and polycytosine (poly C) have been reported to show high affinities to β-1,3-glucans including schizophyllan (SPG).

The application of β-1,3-glucans to RNAi as carriers for siRNAs has been investigated. However, since siRNA is a double-stranded nucleic acid, siRNA itself cannot form a complex with β-1,3-glucan. Thus, a DNA-RNA hetero-nucleic acid, in which a poly(dA) strand is attached to the sense strand of an siRNA for the complexation with a β-1,3-glucan such as SPG is provided which is annealed with the antisense strand of the siRNA to produce a poly(dA)-siRNA, and then, the complexation with SPG undergoes with the aid of the poly(dA) portion.

### Prior Art Literature

### Patent Literature

Patent Literature 1: International Publication No. WO 2001/34207
Patent Literature 2: International Publication No. WO 2002/072152

### Non Patent Literature

Non Patent Literature 1: siRNAs: applications in functional genomics and potential as therapeutics. Y. Dorsett, T. Tuschl, Nat. Rev. Drug Discovery 3 (2004) 318-329.
Non Patent Literature 2: Evaluation of 29-modified oligonucleotides containing 29-deoxy gaps as antisense inhibitors of gene expression. B. Monia, E. Lesnick, C. Gonzalez, W. Lima, D. McGee, C. Guinosso, A. Kawasaki, P. Cook, S. Freier, J. Biol. Chem. 268 (1993) 14514-14522.
Non Patent Literature 3: Potent gene-specific inhibitory properties of mixed-backbone antisense oligonucleotides comprised of 2'-deoxy-2'-fluoro-D-arabinose and 2'-deoxyribose nucleotides. C.N. Lok, E. Viazovkine, K. L. Min, C. J. Wilds, M. J. Damha, M. A. Parniak, Biochemistry 41 (2002) 3457-3467.
Non Patent Literature 4: 2'-O,4'-C-ethylene-bridged nucleic acids (ENA): highly nuclease-resistant and thermodynamically stable oligonucleotides for antisense drug. K. Morita, C. Hasegawa, M. Kaneko, S. Tsutsumi, J. Sone, T. Ishikawa, T. Imanishi and M. Koizumi, Med. Chem. Lett., 12, 73-76 (2002)
Non Patent Literature 5: Human gene therapy comes of age. A.D. Miller, Nature, 357, 455-460 (1992)
Non Patent Literature 6: The basic science of gene therapy. R.C. Mulligan, Science, 14, 926-932 (1993)
Non Patent Literature 7: Controllable gene therapy pharmaceutics of non-viral gene delivery systems. E. Tomlinson and A. P. Rolland, J. Control Release, 39, 357-372
(1996) Non Patent Literature 8: Breathing Life into Polycations: Functionalization with pH-Responsive Endosomolytic Peptides and Polyethylene Glycol Enables siRNA Delivery. M.Meyer, A. Philipp, R. Oskuee, C. Schmidt and E. Wagner, J. Am. Chem. Soc., 130, 3272-3273 (2008)
Non Patent Literature 9: RNA interference-mediated gene silencing of pleiotrophin through polyethylenimine-complexed small interfering RNAs in vivo exerts antitumoral effects in glioblastoma xenografts. M. Grzelinski, B. Urban-Klein, T. Martens, K. Lamszus, U. Bakowsky, S. Hobel, F. Czubayko and A. Aigner, Hum. Gene. Ther., 17, 751-766 (2006)
Non Patent Literature 10: Monomolecular Assembly of siRNA and Poly(ethylene glycol)-Peptide Copolymers. J. DeRouchey, C.Schmidt, G. F. Walker, C. Koch, C. Plank, E. Wagner and J. O. Raedler, Biomacromolecules, 9, 724-732 (2008)
Non Patent Literature 11: Tat-conjugated PAMAM dendrimers as delivery agents for antisense and siRNA oligonucleotides. H. Kang, R. DeLong, M.H. Fisher and R.L. Juliano, Pharm. Res., 22, 2099-2106 (2005)
Non Patent Literature 12: Molecular Recognition of Adenine, Cytosine, and Uracil in a Single-Stranded RNA by a Natural Polysaccharide: Schizophyllan. K. Sakurai and S. Shinkai, J. Am. Chem. Soc., 122, 4520-4521 (2000)
Non Patent Literature 13: Polysaccharide-Polynucleotide Complexes. 2. Complementary Polynucleotide Mimic Behavior of the Natural Polysaccharide Schizophyllan in the Macromolecular Complex with Single-Stranded RNA and DNA. K. Sakurai, M. Mizu and S. Shinkai, Biomacromolecules, 2, 641-650 (2001)
Non Patent Literature 14: Dectin-1 targeting delivery of TNF-α antisense ODNs complexed with β-1,3-glucan protects mice from LPS-induced hepatitis. S. Mochizuki, K. Sakurai, J. Control. Release, 151, (2011) 155-161.
Non Patent Literature 15: Structural Analysis of the Curdlan/Poly (cytidylic acid) Complex with Semiempirical Molecular Orbital Calculations. K. Miyoshi, K. Uezu, K. Sakurai and S. Shinkai, Biomacromolecules, 6, 1540-1546 (2005)

### Disclosure of the Invention

### Problem to be Solved by the Invention

However, DNA-siRNA nucleic acid conjugates have a problem that DNA-siRNA nucleic acid conjugates are prone to be degradated *in vivo* as well as RNA is because DNA-siRNA nucleic acid conjugates are sensitive to ribonucleases widely distributed *in vivo.* In fact, in the observation of the change in molecular weight of a DNA-siRNA nucleic acid conjugate after incubated in 10% serum using acrylamide gel electrophoresis to evaluate the serum stability of the nucleic acid conjugate bands corresponding to degradation product of the nucleic acid conjugate in which cleavage of a bond took place at a bonding site between the DNA and the siRNA were observed. Such cleavage is also observed in a nucleic acid conjugate complexed with β-1,3-glucan. Nucleic acid conjugates introduced in a living body are quickly cleaved to DNA and RNA, which may cause a reduced efficiency of introduction of DNA-siRNA nucleic acid conjugates into cells and a reduced activity to silence target genes.

The present disclosure is completed in view of the above-described problem. An object of the present disclosure is to provide a nucleic acid conjugate which is not degraded at a bonding site between DNA and RNA *in vivo.*

### Means for Solving the Problem

The first aspect of the present disclosure is to solve the above-described problem by providing a nucleic acid conjugate comprising a single-stranded DNA having nucleotides not less than 10 and a double-stranded RNA, wherein the 3' position of the 3'-terminal deoxyribonucleotide residue of the single-stranded DNA and the 5' position of the 5'-terminal ribonucleotide residue of one of the ribonucleotide strands of the double-stranded RNA are bound, and the hydroxyl group at the 2' position of the 5'-terminal nucleotide of the ribonucleotide strand, which is bound to the single-stranded DNA, is substituted with an alkoxy group or a fluorine atom.

The second aspect of the present disclosure is to solve the above-described problem by providing a nucleic acid conjugate comprising a single-stranded DNA having nucleotides not less than 10 and a double-stranded RNA, wherein the 3' position of the 3'-terminal deoxyribonucleotide residue of the single-stranded DNA and the 5' position of the 5'-terminal ribonucleotide residue of one of the ribonucleotide strands of the double-stranded RNA are bound, and the phosphate diester group between the 3' position of the first ribonucleotide bound to the single-stranded DNA and the 5' position of the adjacent ribonucleotide is substituted with any ofphosphorothioate group (thiophosphate ester group: -O-PO(S)-O-, which has a structure formed by substituting a P=S for the P=O of a phosphate group), dithiophosphate diester group (-O-PS(S)-O-) and trithiophosphate diester group (-O-PS(S)-S-).

In the first and the second aspects of the present disclosure, the number of nucleotides in the single-stranded DNA is not less than 10. Moreover, in the first and the second aspects of the present disclosure, the single-stranded DNA may be polydeoxyadenine.

In the first and the second aspects of the present disclosure, at least a part of the phosphate diester groups of the single-stranded DNA may be substituted with any of phosphorothioate group, dithiophosphate diester group and trithiophosphate diester group. Moreover, in this case, at least 50% or more of the phosphate diester groups of the single-stranded DNA may be substituted with any of phosphorothioate group, dithiophosphate diester group and trithiophosphate diester group.

In the first and the second aspects of the present disclosure, the double-stranded RNA may be an siRNA. Moreover, in this case, the siRNA may be a 21-mer or 27-mer siRNA. Furthermore, one or more target genes of the siRNA may be genes expressed in Dectin-1-expressing cells.

The third aspect of the present disclosure is to solve the above-described problem by providing a nucleic acid-polysaccharide complex, wherein the single-stranded DNA portion of one molecule of the nucleic acid conjugate according to the first and the second aspects of the present disclosure and two molecules of a polysaccharide having a β-1,3-glucan backbone form a triple helix structure.

In the third aspect of the present disclosure, the polysaccharide having a β-1,3-glucan backbone is preferably schizophyllan.

The fourth aspect of the present disclosure is to solve the above-described problem by providing a pharmaceutical composition comprising the nucleic acid-polysaccharide complex according to the third aspect of the present disclosure.

The fifth aspect of the present disclosure is to solve the above-described problem by providing a method of enhancing the stability of a nucleic acid conjugate to a ribonuclease, wherein the nucleic acid conjugate comprises a single-stranded DNA having nucleotides not less than 10 and a double-stranded RNA; and the 3' position of the 3'-terminal deoxyribonucleotide residue of the single-stranded DNA and the 5' position of the 5'-terminal ribonucleotide residue of one of the ribonucleotide strands of the double-stranded RNA are bound; the method comprising substituting the hydroxyl group at the 2' position of the 5'-terminal nucleotide of the ribonucleotide strand, which is bound to the single-stranded DNA, by an alkoxy group or a fluorine atom to stabilize the nucleic acid conjugate.

The sixth aspect of the present disclosure is to solve the above-described problem by providing a method of enhancing the stability of a nucleic acid conjugate to a ribonuclease, wherein the nucleic acid conjugate comprises a single-stranded DNA having nucleotides not less than 10 and a double-stranded RNA; and the 3' position of the 3'-terminal deoxyribonucleotide residue of the single-stranded DNA is bound to the 5' position of the 5'-terminal ribonucleotide residue of one of the ribonucleotide strands of the double-stranded RNA; the method comprising substituting the phosphate diester group between the 3' position of the first ribonucleotide bound to the single-stranded DNA and the 5' position of the adjacent ribonucleotide by any of phosphorothioate group, dithiophosphate diester group and trithiophosphate diester group to stabilize the nucleic acid conjugate.

### Effects of the Invention

In a DNA-RNA nucleic acid conjugate comprising a single-stranded DNA having nucleotides not less than 10 and a double-stranded RNA, in which the 3' position of the 3'-terminal deoxyribonucleotide residue of the single-stranded DNA and the 5' position of the 5'-terminal ribonucleotide residue of one of the ribonucleotide strands of the double-stranded RNA are bound, the stability of a bonding site between the polydeoxyribonucleotide strand and the polyribonucleotide strand is improved and the bonding site is hardly hydrolyzed even *in vivo* by substituting the hydroxyl group at the 2' position of the 5'-terminal nucleotide of the ribonucleotide strand, which is linked to the single-stranded DNA, with an alkoxy group or a fluorine atom, or alternatively by substituting the phosphate diester group between the 3' position of the first ribonucleotide linked to the single-stranded DNA and the 5' position of the adjacent ribonucleotide with any of phosphorothioate group, dithiophosphate diester group and trithiophosphate diester group. Thus, a nucleic acid conjugate of the present disclosure can maintain the intrinsic function without undergoing quick degradation *in vivo,* which leads to loss of function of the nucleic acid conjugate. Applying a nucleic acid conjugate of the present disclosure, for example, to a nucleic acid-polysaccharide complex formed of β-1,3-glucan strand and a nucleic acid conjugate, in which a polydeoxynucleotide strand such as poly(dA) strand, which interacts with the β-1,3-glucan (schizophyllan and the like) strand to form a stable complex, is bound to the 5' terminus of an siRNA, allows the siRNA to be protected from degradation in cytoplasm and surely to be delivered to cell nuclei, thus one can expect that the usability and therapeutic effect of nucleic acid medicines which suppress expression of genes responsible for diseases by means of RNA interference may be improved.

### Brief Description of Drawings

FIG. 1 represents a fluorescence image of a gel showing the result of the degradation experiment on dA40-siRNA in serum (Example 1);
FIG. 2A represents fluorescence images of a gel showing the results of the identification experiment for serum degradation products of a fluorescence-labeled dA10-siRNA prepared by using an antisense strand ' labeled with FITC at 5'-terminal (Example 2);
FIG. 2B represents fluorescence images of a gel showing the results of identification experiment for serum degradation products of a fluorescence-labeled dA10-siRNA prepared by using an antisense strand ' labeled with FITC at 3'-ternimal (Example 2);
FIG. 2C represents a schematic drawing showing the degradation reaction of the dA-siRNA;
FIG. 3 represents a fluorescence image of a gel showing the effect of the '2'-O-methylation on degradation of RNA (Example 3);
FIG. 4 represents a fluorescence image of a gel showing the effect of the '2'-O-methylation on degradation of single-stranded RNA (Example 4);
FIG. 5 represents a fluorescence image of a gel showing the effect of the position of the '2'-O-methylation on degradation of nucleic acid conjugate in serum (Example 5);
FIG. 6 represents a fluorescence image of a gel showing the influence of the type of a base on degradation of DNA (Examples 6 and 7);
FIG. 7 represents a fluorescence image of a gel showing the effect of the phosphorothioate modification on degradation of RNA (Example 8); and
FIG. 8 represents a fluorescence image of a gel showing the influence of DNA bound to the 3' terminus of RNA (Example 9).

### Mode for Carrying Out the Invention

Next, modes for carrying out the present disclosure will be described below.

A nucleic acid conjugate according to the first embodiment of the present disclosure is a nucleic acid conjugate comprising a single-stranded DNA having nucleotides not less than 10 and a double-stranded RNA, wherein the 3' position of the 3'-terminal deoxyribonucleotide residue of the single-stranded DNA and the 5' position of the 5'-terminal ribonucleotide residue of one of the ribonucleotide strands of the double-stranded RNA are bound, and the hydroxyl group at the 2' position of the 5'-terminal nucleotide of the ribonucleotide strand, which is bound to the single-stranded DNA, is substituted with an alkoxy group or a fluorine atom. In other words, the nucleic acid conjugate according to the present embodiment corresponds to a general formula (I) below, wherein R² represents an alkoxy group or a fluorine atom. Additionally, in the formula (I), R¹ represents any of adenine (A), guanine (G), uracil (U) and cytosine (C), and R³ and R⁵ represent phosphate diester group (-PO₂-O-). Additionally, specific examples of the alkoxy group include such as a linear or branched alkoxy group having 1-5 carbon atoms, an arylalkyl group having 5-15 carbon atoms, and an alkenylalkyl group such as O-allyl group, and the like, and an alkoxy group having 1-3 carbon atoms is preferable, and methoxy group is especially preferable.

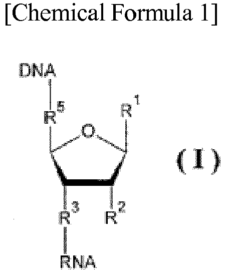

For example, in the nucleic acid conjugate, a polyribonucleotide portion bound to a polydeoxynucleotide portion may form a double-stranded RNA with an RNA strand having a complementary base sequence to form an siRNA. siRNA is a class of short double-stranded RNA that is involved in a phenomenon called RNA interference (RNAi) and has a function to destruct mRNA molecules comprising a base sequence complementary to that of an siRNA and thereby to suppress gene expression in a sequence-specific manner.

The siRNA comprises 20-27 bases (base pairs). In a case of human, when the number of base pairs is not less than 17, the total number of polynucleotide species to be obtained (4¹⁷ = 1.7 x 10¹⁰) is larger than the total number of genes in human (6 x 10⁹) and, therefore, inhibiting only particular genes is statistically feasible. The siRNA may be a 21-mer siRNA, which is broadly used, or a 27-mer siRNA, having a further improved specificity for Dicer.

A base sequence can be designed for the siRNA by any known method. Because selecting a sequence complementary to a sequence highly conserved among multiple genes can sometimes cause difficulties in suppression of expression in a gene-specific manner, a sequence complementary to, for example, a target gene-specific sequence is selected. In cases where a target base sequence or an amino acid sequence corresponding to a gene product is known, siRNA can be designed based on the known sequence data, which is available from a database such as GenBank, EMBL, PDB, DDBJ and the like, by any known method.

The single-stranded DNA (polydeoxynucleotide) portion in the nucleic acid conjugate itself may have an intrinsic function, may improve the stability of the double-stranded RNA (polyribonucleotide) portion, or may have a particular base sequence (including a repetitive sequence) to enhance the ability to form a complex in cases where the complex (nucleic acid-polysaccharide complex) is formed of the nucleic acid conjugate and β-1,3-glucan, as described below. The number of nucleotides in the single-stranded DNA is not less than 10. In order to improve the stability against nucleases, a part, more preferably 50% or more, of the phosphate diester groups (also referred to as phosphate diester bonds, phosphodiester bonds, and the like), namely, R⁵ in the above-described general formula (I), in the single-stranded DNA may be substituted with any of phosphorothioate group (thiophosphate ester group: -O-PO(S)-O-, which has a structure formed by substituting a P=S for the P=O of a phosphate group), dithiophosphate diester group (-O-PS(S)-O-) and trithiophosphate diester group (-O-PS(S)-S-).

A polynucleotide strand having a base sequence as described above can be synthesized by any known method such as a chemical synthesis method, a genetic engineering procedure, and the like.

A nucleic acid conjugate according to the second embodiment of the present disclosure is a nucleic acid conjugate comprising a single-stranded DNA and a double-stranded RNA, wherein the 3' position of the 3'-terminal deoxyribonucleotide residue of the single-stranded DNA and the 5' position of the 5'-terminal ribonucleotide residue of one of the ribonucleotide strands of the double-stranded RNA are bound, and the phosphate diester group between the 3' position of the first ribonucleotide bound to the single-stranded DNA and the 5' position of the adjacent ribonucleotide is substituted with any of phosphorothioate group, dithiophosphate diester group and trithiophosphate diester group. In other words, the nucleic acid conjugate according to the present embodiment corresponds to a general formula (I) below, wherein R³ represents any of phosphorothioate group, dithiophosphate diester group and trithiophosphate diester group. Additionally, in the formula (I), R¹ represents any of adenine (A), guanine (G), uracil (U) and cytosine (C), R² represents hydroxyl group, and R⁵ represents phosphate diester group (-PO₂-O-).

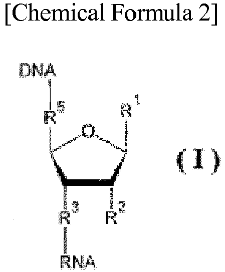

Additionally, in the above-described general formula (I), R² may be an alkoxy group or a fluorine atom, and R⁵ may be any of phosphorothioate group, dithiophosphate diester group and trithiophosphate diester group.

A nucleic acid conjugate, which is obtained as described above, interacts with β-1,3-glucan strand to form a nucleic acid-polysaccharide complex, in which the single-stranded DNA portion of one molecule of the nucleic acid conjugate and two molecules of a polysaccharide having a β-1,3-glucan backbone form a triple helix structure. Upon forming a complex of polynucleotide and β-1,3-glucan strands, polynucleotide can be protected against hydrolysis and the half-life of the polynucleotide can be significantly prolonged (for example, around ten-fold longer) in blood and body fluid and, therefore, a nucleic acid conjugate comprising an siRNA portion, for example can be surely delivered nucleic acid conjugate to target cells.

It is known that polysaccharides whose main strands are composed of β-1,3-glucan and β-1,3-xylan have helix parameters similar to those of nucleic acids such as poly(C) (see, for example, Takahashi, Obata, and Suzuki. Prog. Polym. Phys. Jpn. 27: pp 767, and "Conformation of Carbohydrates," Sharwood academic publisher, 1998) and have hydroxyl groups that can form hydrogen bonds with nucleobases and, therefore, interact with nucleic acid to form a stable complex having a triple helix structure. Specific examples of β-1,3-glucan include schizophyllan, curdlan, lentinan, pachyman, gliforan, scleroglucan, and the like. These are naturally occurring polysaccharides called glucans whose main chains are composed of units connected by β-bonds β-D-bonds) and whose side chains appear in different frequencies. These β-1,3-glucans may be used without any change due to treatment such as chemical modification, or can be subjected to a regular periodic acid treatment to remove some of the side chains in an appropriate manner and thereby control the solubility thereof

The molecular weight of a β-1,3-glucan strand is appropriately adjusted depending on the base length of a polynucleotide strand used for preparation of a therapeutic agent for inflammatory bowel disease, the base length of a repetitive sequence, and the like. However, when the molecular weight is low, the so-called cluster effect (a cooperative phenomenon in the macromolecular system) is hardly exhibited, which is not preferable. The weight-average molecular weight of a β-1,3-glucan strand that can form a complex with a nucleic acid fragment is normally variable depending on the types and the conformation of the nucleobases contained in the nucleic acid fragment and is preferably not less than 2000, further preferably not less than 4000, and more preferably not less than 6000. Moreover, the number of hydroxyl groups required to form hydrogen bonds with nucleobases on the polynucleotide strand is normally not less than 5, preferably not less than 8, and further preferably not less than 10.

The weight-average molecular weight of a β-1,3-glucan strand can be determined using any known method such as light scattering method, sedimentation velocity method (ultracentrifugal method), and the like.

β-1,3-glucans are generally produced by fungi and bacterium and can accordingly be obtained by culturing these microorganisms and subsequently homogenizing fungal or bacterial bodies, which is followed by isolation from impurities such as cell extract or insoluble residues by a method including ultracentrifugation and the like. Normally, β-1,3-glucans obtained in such a process have high molecular weights (a weight-average molecular weight of around several hundred thousand) and have a triple helix structure and may be used without any modification or may be used with reduction of molecular weight if necessary. The molecular weight is reduced by a method and conditions suitable for a β-1,3-glucan, which are appropriately selected depending on the type of the β-1,3-glucan and a desired molecular weight from any methods including enzymatic degradation methods, acid hydrolysis methods and the like and any conditions for those methods. For example, in a case of schizophyllan, single-stranded schizophyllan molecules having various molecular weights can be obtained by a hydrolysis method using sulfuric acid in 80% DMSO, and the like.

β-1,3-glucans such as schizophyllan normally adopts a triple helix structure in water and, accordingly, are dissolved in a solvent such as DMSO (dimethylsulfoxide) for unfolding the association state through intermolecular hydrogen bonding and hydrophobic interaction to single-strand molecules in order to form a complex with one or more polynucleotide strands. Upon adding an aqueous solution (or solution of a polar solvent such as alcohol) containing a polynucleotide strand to such a solution, a β-1,3-glucan strand is associated with the polynucleotide strand through hydrophobic interaction as the polarity of the solvent increases and forms an assembly of polynucleotide and polysaccharide through intramolecular and intermolecular association while incorporating molecules of the polynucleotide strand. Consequently, a complex having a triple helix structure consisting of one molecule of the polynucleotide molecule and two molecules of the β-1,3-glucan molecule is formed. Formation of a complex can be confirmed by examining the conformational change through, for example, CD (circular dichroism) spectroscopic measurement. The obtained complex is normally soluble in water and is dissociated and reassociated depending on the change in temperature or pH. Furthermore, the complex is resistant to nucleases and, furthermore, the polynucleotide portion is not destructed.

For forming a nucleic acid-polysaccharide complex as described above, a single-stranded DNA portion of the nucleic acid conjugate preferably has a repetitive sequence of either poly(dA) sequence or poly(dT) sequence in order to improve the complex forming ability. The kinds of bases and nucleotides and the number of bases that constitute a preferred repetitive sequence are appropriately determined depending on the length of a ribonucleotide portion, the type of a β-1,3-glucan to be used and the molecular weight of a β-1,3-glucan strand to be used. For example, in cases where schizophyllan is used as a β-1,3-glucan, a polydeoxynucleotide portion preferably has a poly(dA) sequence as a repetitive sequence and the length of the repetitive sequence is not less than 10 bases, and more preferably in a range of 10-80 bases.

Any known methods can be used without any particular limitation to evaluate the serum stability of the nucleic acid conjugate. One example is a method, in which fetal bovine serum (FBS) is selected as a type of serum to be used, RPMI1640 medium is prepared so as to include 10% FBS, to which DNA-siRNA nucleic acid conjugate is added and the resultant mixture is incubated at 37°C for 1 hour and is electrophoresed through a 12% acrylamide gel, which is followed by staining of the nucleic acid with SYBR Gold and observation using a gel imaging apparatus.

The nucleic acid-polysaccharide complex can be used as an active ingredient in production of pharmaceutical compositions for gene therapies including RNAi. Any known ingredients (any pharmaceutically acceptable carriers, excipients and additives) and formulation methods can be used in production of the pharmaceutical compositions. For example, a therapeutic agent for inflammatory bowel disease can have the form of tablets, suppositories, capsules, syrups, microcapsules made of nanogel and the like, sterilized solutions, suspensions, and the like.

The pharmaceutical compositions can be administered to human or warm-blooded animals (mouse, rat, rabbit, sheep, pig, cow, horse, chicken, cat, dog, monkey, and the like) via either oral or parenteral route. Examples of parenteral administration routes include subcutaneous and intramuscular injections, intraperitoneal administration, rectal administration, enteral administration through an endoscope or the like, and the like.

A dose of a complex of a nucleic acid conjugate and a β-1,3-glucan molecule as an active ingredient will vary depending on the activity of the complex, the disease to be treated, the type of an animal as a subject of administration, body weight, gender, age, the severity of the disease, mode of administration, and the like. In the example of an adult human having a body weight of 60 kg, a daily dose in oral administration is typically in a range of about 0.1 to about 100 mg, preferably of about 1.0 to about 50 mg, and more preferably of about 1.0 to about 20 mg, and a daily dose in parenteral administration is typically in a range of about 0.01 to about 30 mg, preferably of about 0.1 to about 20 mg, and more preferably of about 0.1 to about 10 mg. In case of administration to other animals, the above-described dose is converted to a dose per unit body weight, which is subsequently multiplied with the body weight of an animal as a subject of administration and the resulting dose is used.

In cases where the nucleic acid-polysaccharide complex is used as an active ingredient of pharmaceutical compositions, a base sequence of the nucleic acid conjugate is appropriately selected depending on the disease to be treated and the type of a target gene and preferably comprises the base sequence of an siRNA which targets one or more genes expressed in Dectin-1-expressing cells. Dectin-1 is a membrane protein belonging to C-type lectin family, which is expressed in dendritic cells and macrophages and has a binding property to β-glucans and, therefore, is suitable for specific introduction of a nucleic acid-polysaccharide complex.

Nucleic acid conjugates which may be used in production of a nucleic acid-polysaccharide complex (including the nucleic acid conjugates according to the first and the second embodiments as described above) have the partial base sequence represented by the formula (A) below. The nucleic acid conjugate may constst of the base sequence represented by formula (A) alone, or may have the base sequence as a partial base sequence.

[Chemical Formula 3]

**5'- (dRN)ₓ - (AN)_{y} - (RN)_{z} -3'** **(A)**

In the formula (A), dRN represents a deoxyribonucleotide, AN represents any of a ribonucleotide derivative in which one or both of the hydroxy group at the 2' position and the phosphate diester group at the 5' position of a ribonucleotide are chemically modified, peptide nucleic acid (PNA), glycol nucleic acid (GNA), locked nucleic acid (LNA), threose nucleic acid (TNA) and morpholino nucleic acid, RN represents a ribonucleotide, each of x and z independently represents an integer of not less than 1, and y represents an integer of not less than 1 and not more than 10.

The polydeoxynucleotide portion (dRN)ₓ locating at the 5'-terminal side in the base sequence represented by the formula (A) is represented by the formula (III) below, wherein the base B¹ is adenine (A), guanine (G), thymine (T) or cytosine (C). Moreover, the polyribonucleotide portion (RN)_{z} locating at the 3'-terminal side in the base sequence represented by the formula (I) is represented by the formula (IV) below, wherein the base B² is adenine (A), guanine (G), uracil (U) or cytosine (C).

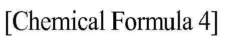

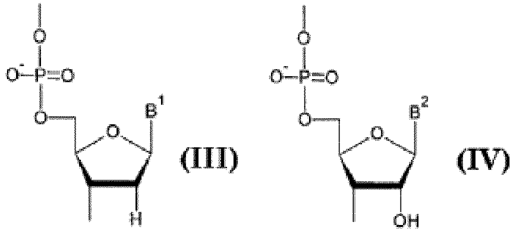

The numbers represented by x and z, which are the numbers of bases comprised in the polydeoxynucleotide portion (dRN)ₓ and the polyribonucleotide portion (RN)_{z}, respectively, are not particularly limited. Moreover, respective base sequences of the polydeoxynucleotide portion (dRN)ₓ and the polyribonucleotide portion (RN)_{z} may be a sequence encoding a gene having some kind of biological function or a part of such gene or a primer sequence, or a sequence having no biological functions, such as a sequence in which a certain number of bases of a single type are arranged, a sequence in which several types of bases are arranged in a regular manner, and the like.

Specific examples of a repetitive unit comprised in the (AN)_{y} locating between the polydeoxynucleotide portion and the polyribonucleotide portion in the base sequence represented by the formula (I) include a substituted ribonucleotide represented by the general formula (II) below, a peptide nucleic acid (PNA) represented by the formula (V) below, a glycol nucleic acid (GNA) represented by the formula (VI) below, a locked nucleic acid (LNA) represented by the formula (VII) below, a threose nucleic acid (TNA) represented by the formula (VIII) below, a morpholino nucleic acid represented by the formula (IX) below, and the like.

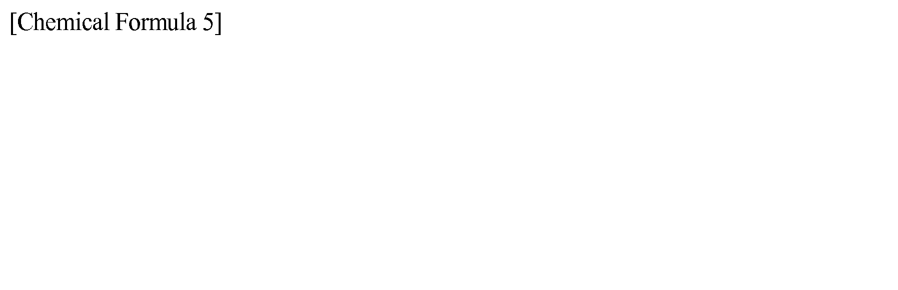

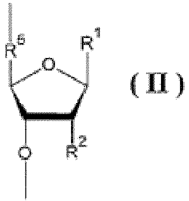

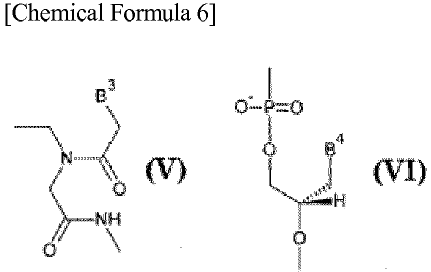

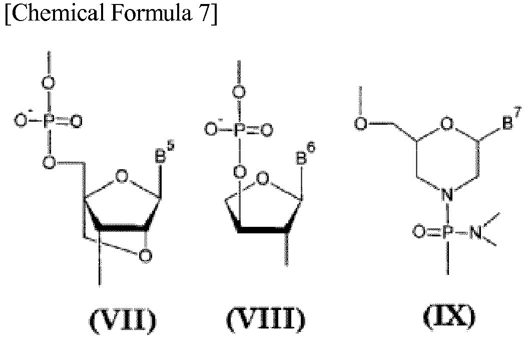

R¹ in the formula (II) and B³ to B⁷ in the formulas (V) to (IX) represent any of adenine (A), guanine (G), cytosine (C), uracil (U) and an unnatural base (examples of the unnatural base include thymine, 8-oxoguanine, 2-amino-6-dimethylaminopurine, 2-amino-6-thienylpurine, pyridin-2-one, 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, β-D-galactosylqueuosine, 2'-O-methylguanosine, inosine, N6-isopentenyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, β-D-mannosylqueuosine, 5-methoxycarbonylmethyl-2-thiouridine, 5-methoxycarbonylmethyluridine, 5-methoxyuridine, 2-methylthio-N6-isopentenyladenosine, N-((9-β-D-ribofuranosyl-2-methylthiopurine-6-yl)carbamoyl)threonine, N-((9-β-D-ribofuranosylpurine-6-yl)N-methylcarbamoyl)threonine, uridine-5-oxyacetic acid-methylester, uridine-5-oxyacetic acid, wybutoxosine, pseudouridine, queuosine, 2-thiocytidine, 5-methyl-2-thiouridine, 2-thiouridine, 4-thiouridine, 5-methyluridine, N-((9-β-D-ribofuranosylpurine-6-yl)carbamoyl)threonine, 2'-O-methyl-5-methyluridine, 2'-O-methyluridine, wybutosine, 3-(3-amino-3-carboxypropyl)uridine and the like).

In the formula (II), R² represents any of hydrogen atom (H), a fluorine atom, hydroxyl group, a linear or branched alkoxy group having 1-5 carbon atoms, an arylalkyl group having 5-15 carbon atoms, and an alkenylalkyl group such as O-allyl group, and R⁵ represents any of phosphate diester group, phosphorothioate group (thiophosphate diester group), dithiophosphate diester group, and trithiophosphate diester group (however, excluding a case in which R² is a hydroxyl group and R⁵ is a phosphate diester group).

Out of repetitive units for the (AN)_{y} portion, especially preferable is a repetitive unit in which R² is an O-methyl group and R⁵ is a phosphate group, or R² is a hydroxyl group and R⁵ is a phosphorothioate group, in the formula (II).

The base sequence of the (AN)_{y} portion may comprise a part of a base sequence encoding a gene having a biological function, which base sequence is continuously shared with the polydeoxyribonucleotide portion and/or the polyribonucleotide portion, may have a base sequence having some type of function in an independent manner, or may be a sequence in which bases are arranged regularly or randomly and which sequence does not have a particular function. y represents an integer of not less than 1 and not more than 10, preferably of not less than 1 and not more than 4, and especially preferably 1.

### Examples

Next, Examples which were performed to confirm the effects of the present disclosure will be described.

### Example 1: Confirmation of degradation of a (dA)₄₀-siRNA in serum.

A DNA-RNA nucleic acid conjugate, in which 40-mer of deoxyadenines (hereinafter sometimes abbreviated as "dA") (dA40) is attached to the 5' terminus of the sense strand of an siRNA (5'-CAAAGACAACCAACUAGUGGU-3': SEQ ID NO: 1; hereinafter referred to as "the siRNA," "the sense strand of the siRNA" or "the sense strand"), was annealed with the antisense strand of the siRNA (5'-ACCACUAGUUGGUUGUCUUUG-3': SEQ ID NO: 2) to obtain a DNA-siRNA nucleic acid conjugate (dA40-siRNA; hereinafter abbreviated as "dAx-siRNA" is a DNA-siRNA nucleic acid conjugate obtained by annealing a DNA-RNA nucleic acid conjugatein which nucleic acid conjugatex-mer (x represents a natural number) of deoxyadenines is attached to the 5' terminus of a sense strand and an antisense strand.).

All the nucleic acids used were purchased from Hokkaido System Science Co., Ltd.

The DNA-siRNA nucleic acid conjugate was added to a RPMI1640 medium containing 10% FBS and incubated at 37°C for 1 hour and electrophoresed through a 12% acrylamide gel (at 100 V for 1 hour). The gel was stained with SYBR Gold (Life Technologies Co., California, USA) and a fluorescence image of the gel was taken with a fluorescence imager.

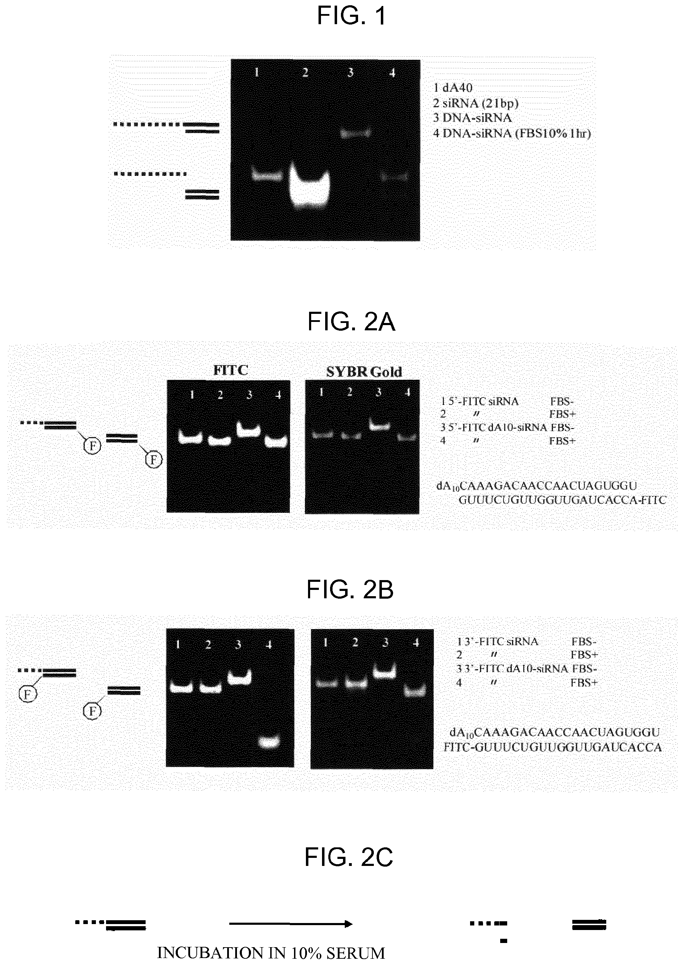

The gel fluorescence image thus obtained is shown in FIG. 1. A band of a dA40-siRNA nucleic acid conjugate before incubation is detected at a position corresponding to a higher molecular weight as compared with bands of the dA40 and the siRNA (21 bp) as control samples. However, in the sample of the DNA-siRNA nucleic acid conjugate after incubation in the FBS-containing medium, two bands were observed at positions corresponding to lower molecular weights than the molecular weight of the DNA-siRNA nucleic acid conjugate before incubation. Judging from the positions of the bands, the DNA-siRNA nucleic acid conjugate is considered to be cleaved to a DNA and an siRNA through the action of an enzyme contained in FBS. A similar result was obtained in a similar experiment performed using a RPMI1640 medium containing mouse serum (MS) instead of FBS.

### Example 2: Examination of a cleavage site in a DNA-siRNA nucleic acid conjugate.

The way of cleavage of a DNA-siRNA nucleic acid conjugate by an enzyme in serum was investigated by using fluorescence-modified nucleic acids. A 'antisense strand modified with FITC at the 5' terminus and a 'antisense strand modified with FITC at the 3' terminus were prepared, respectively, and each of them was annealed with a DNA-RNA nucleic acid conjugate comprising a sense strand having a base sequence represented by SEQ ID NO: 1 and 10-mer of deoxyadenines (dA10) attached to the 5' terminus of the sense strand and with the sense strand to which dA10 was not attached, respectively. Both products were incubated in serum, followed by gel electrophoresis and observation of the FITC fluorescence. Then, the products were stained with SYBR Gold and fluorescence observation was performed similarly.

The fluorescence images thus obtained are shown in FIG. 2A and FIG. 2B. In a case of the siRNA obtained by annealing the sense strand to which dA10 is not attached with the 'antisense strand modified with FITC at the 5' terminus, fluorescent bands appeared in the fluorescence images of FITC and of SYBR Gold staining are found to coincide completely between both the fluorescence images. Moreover, the positions of the fluorescent bands are unchanged in the samples before and after the incubation, which indicates that no significant degradation took place in the double stranded RNA (FIG. 2A, lanes 1 and 2). On the other hand, in a case of dA10-siRNA, comparison of the positions of fluorescent bands detected after staining with SYBR Gold between the samples before and after the incubation showed that the molecular weight of the dA10-siRNA was reduced after the incubation with FBS and that the fluorescent band arising from the SYBR Gold staining overlapped with the fluorescent band arising from the FITC in the sample after the incubation (FIG. 2A, lanes 3 and 4). Furthermore, the positions of these fluorescent bands almost overlap with the position of the fluorescent band for the control siRNA, which indicates the fluorescent band observed after the incubation with serum is arised from a degradation product having a number of base pairs close to that of the siRNA comprising 21 base pairs.

In a case of the siRNA comprising the 'antisense strand modified with FITC at the 3' terminus, the positions of the fluorescent bands derived from the FITC and the SYBR Gold staining are unchanged in the samples before and after the incubation with FBS, as is the case with the 'antisense strand modified with FITC at the 5' terminus described above (FIG. 2B, lanes 1 and 2). This result indicates that the siRNAs are not degraded by incubation in serum regardless of the FITC introduction sites (the 5'-terminal site and the 3'-terminal site). On the other hand, in the dA10-siRNA, a fluorescent band at a position corresponding to a very low molecular weight has been observed after the incubation with FBS (FIG. 2B, lanes 3 and 4). Moreover, in the fluorescence image obtained after staining with SYBR Gold, the siRNA was observed at a position close to 21 bp, as in the case described above. From this result, the obtained double-stranded RNA has a length shorter than 21 bp, which shows that not only the sense strand but also the antisense strand were cleaved by an enzyme protein in FBS.

The above-described results suggest that the position on the RNA strand around the bonding site between the RNA strand and the DNA strand in the dA10-siRNA is recognized and degraded in serum and that the dA10-siRNA is divided after the degradation into a double-stranded RNA having a length shorter than 21 bp, a single-stranded nucleic acid in which an oligomer of RNA attached to dA, and a RNA strand complementary to the oligomer of RNA (a schematic model is shown in FIG. 2C).

### Example 3: Avoiding a degradation of a DNA-siRNA nucleic acid conjugate 2'-O-methylation of an RNA residue in the siRNA.

Since the results of Example 2 suggest degradation around the bonding site between the DNA and the siRNA, provided are a series of dA40-siRNA nucleic acid conjugates in which the first one, two, three, or four RNA residues from the 5' terminus of a strand of the siRNA (sense strand) bound to the 3' terminus of the DNA (dA40) were 2'-O-methylated, respectively, to evaluate the stability of the nucleic acid conjugates in serum (FBS) as in Example 2 by a electrophoresis method using samples before and after incubation.

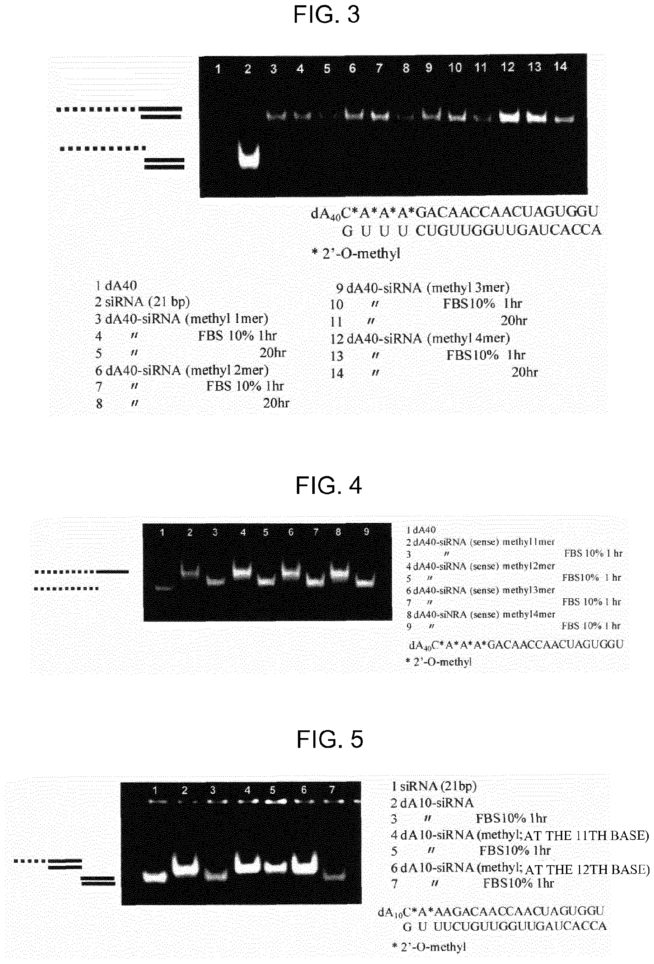

The gel fluorescence image thus obtained is shown in FIG. 3. It was shown that the positions of bands correspoding to the methylated DNA-siRNA nucleic acid conjugate were preserved even after 20 hours of incubation whereas the former unmethylated DNA-siRNA was cleaved by incubation with serum for 1 hour. Moreover, it was found that 2'-O-methylation only at the first RNA residue (at the 5' terminus) bound to the DNA was sufficient to prevent degradation.

### Example 4: Comparison between a DNA-siRNA nucleic acid conjugate and a DNA-RNA nucleic acid conjugate.

The previous experiment suggests that the degradation of the DNA-siRNA nucleic acid conjugate by incubation in serum occurs at the bonding site between the DNA portion and the double-stranded RNA portion. In order to examine whether this degradation is a phenomenon specific for double-stranded RNAs, an experiment similar to that in Example 3 was performed using a series of (single-stranded) dA40-RNA nucleic acid conjugates in which the first one, two, three, or four RNA residues from the 5' terminus of a strand of the siRNA (sense strand) bound to the 3' terminus of the DNA (dA40) were 2'-O-methylated, respectively.

The gel fluorescence image thus obtained is shown in FIG. 4, which indicates that the molecular weights of all the samples are reduced after the incubation with FBS regardless of the number of methylated RNA residues. On the other hand, the dA40 portion is not significantly degraded in 1 hour, which suggests therefore that the reduction in molecular weight is caused by degradation of the single-stranded RNA portion (which is an unmethylated portion).

In a case of the double-stranded RNA, 2'-O-methylation of the first RNA residue from the 5' terminus of the RNA sense strand bound to the 3' terminus of the DNA was sufficient to avoid degradation but, in a case of the single-stranded RNA, the RNA portion excluding one or more 2'-O-methylated residues was found to be degraded even though one or more RNA residues inside the RNA portion bound to the 3' terminus of the DNA portion were 2'-O-methylated. This result showed that 2'-O-methylation of an RNA residue adjacent to the 3' terminus of a DNA strand was effective for a DNA-siRNA nucleic acid conjugate to avoid the degradation but not for a DNA-RNA nucleic acid conjugate.

### Example 5: Examination of the position of an RNA residue for 2'-O-methylation effective in avoidance of degradation of a DNA-siRNA nucleic acid conjugate.

The result of Example 3 indicated that 2'-O-methylation of one RNA residue 'was sufficient for preventing degradation of the DNA-siRNA nucleic acid conjugate. In order to investigate whether the 2'-O-methylation site was required to be the first RNA residue from the 5' terminus of the sense strand bound to the 3' terminus of the DNA, a similar experiment was performed, using the DNA-siRNA nucleic acid conjugate in which the position of an RNA residue to be 2'-O-methylated was changed. The 5'-terminal RNA residue or the second RNA residue from the 5' terminus was 2'-O-methylated. Moreover, since the length of dAs was 40-mer in view of complexation with SPG in the above-described Example, it was also investigated whether a similar phenomenon was observed even in the DNA-siRNA sample having shorter dA portion.

The gel fluorescence image thus obtained is shown in FIG. 5. A band corresponding to shorter species (having a length similar to that of the siRNA) was observed after the incubation with FBS even though the length of dA was shortened to 10-mer (FIG. 5, lane 3). Moreover, in the sample in which the position of an RNA residue to be 2'-O-methylated was changed, it was found that the degradation in serum was not able to avoid by the methylation of the second RNA residue from the 5' terminus (FIG. 5, lane 7).

The above-described result indicates that the first RNA residue bound to a DNA strand is required to be 2'-O-methylated in order to prevent degradation of a DNA-siRNA nucleic acid conjugate.

### Example 6: Investigation of the serum stability of a DNA-dsDNA nucleic acid conjugate.

It is a DNA-double-stranded RNA nucleic acid conjugate that was confirmed in the above-described Examples to have undergone degradation in serum (FBS). In order to examine whether a similar degradation was observed even in a case of a DNA-double-stranded DNA (dsDNA) nucleic acid conjugate, DNA strands that adopted the whole sequences of the siRNA strands (both sense and antisense strands) were provided and a similar experiment was performed.

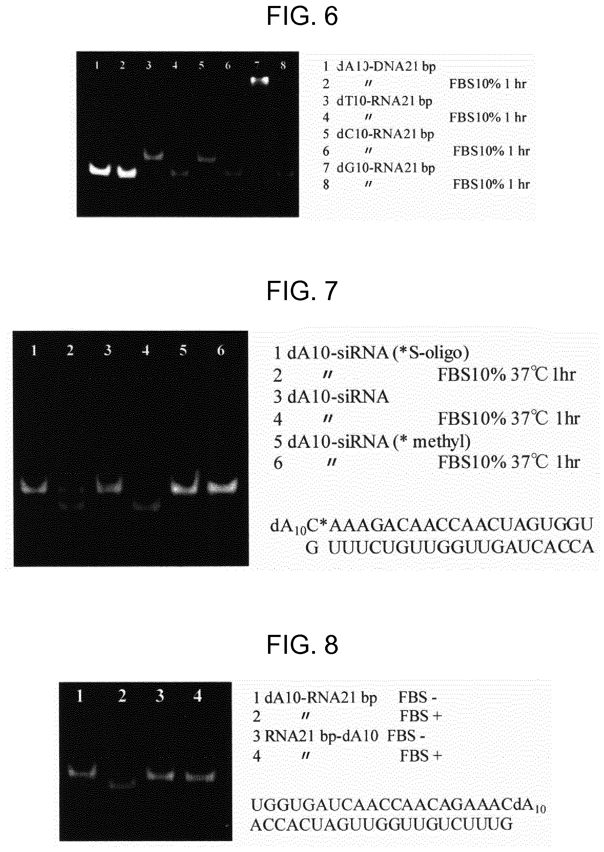

The obtained gel fluorescence image is shown in FIG. 6, which indicates that the molecular weight is unchanged before and after the incubation with serum in the DNA-dsDNA nucleic acid conjugate (FIG. 6, lanes 1 and 2). This result indicates that only a double-stranded RNA in a nucleic acid conjugate undergoes degradation.

### Example 7: Relationship between a base sequence of the DNA in a DNA-siRNA nucleic acid conjugate and the stability of the DNA-siRNA nucleic acid conjugate.

In the previous experiments, dA10 or dA40 has been consistently used as a DNA portion in view of complexation with SPG. In order to examine the influence of the base sequence of a DNA portion on the stability of a DNA-siRNA nucleic acid conjugate, 10-mers of deoxycytosine (dC), deoxyguanine (dG), and deoxythymidine (dT) were used instead of dA10 to prepare nucleic acid conjugates with an siRNA, respectively, and a similar experiment was performed.

The gel fluorescence image thus obtained is shown in FIG. 6. The reduction of molecular weight (a fluorescent band corresponding to the molecular weight similar to that of the siRNA alone) was observed in a dT10-siRNA and a dC10-siRNA after the incubation with FBS as in the case of the dA10-siRNA, which suggests that those nucleic acid conjugates were cleaved on the siRNA portion similarly. In a dG10-siRNA, the sample before the incubation showed a fluorescence band at a position corresponding higher molecular weight compared with other DNA-siRNA nucleic acid conjugate samples because it tends to aggregate at the dG10 portion, while a fluorescent band was observed after the incubation with FBS at a position similar to that of the siRNA. These results indicate that the degradation also takes place in the dG10-siRN nucleic acid conjugate similarly.

### Example 8: Avoiding the degradation of a DNA-siRNA nucleic acid conjugate by a chemical modification other than 2'-O-methylation.

It was found that the degradation of the DNA-siRNA nucleic acid conjugate in serum may be avoided by '2'-O-methylation (substitution of the hydroxyl group at the 2' position of a ribose with a methoxy group) of the first ribonucleotide bound to a DNA strand (a ribonucleotide adjacent to the 3' terminus of a DNA strand) As a DNA-siRNA nucleic acid conjugate other than 2'-O-methylated DNA-siRNA nucleic acid conjugate, a nucleic acid conjugate in which the phosphate diester group between the 3' position of the first ribonucleotide bound to the 3' terminus of a DNA strand and the 5' position of the second ribonucleotide is substituted with a phosphorothioate group was provided and a similar experiment was performed.

The gel fluorescence image thus obtained is shown in FIG. 7. In DNA-siRNA nucleic acid conjugate having the sequence in which phosphate diester groups are substituted with phosphorothioate groups, approximately half of the nucleic acid conjugate molecules remained intact, while the rest of the nucleic acid conjugate molecules was degraded. A phosphorothioate derivative is a compound in which one oxygen atom bound to the phosphate group in a phosphodiester backbone is substituted with a sulfur atom and is obtained as a racemic mixture. Accordingly, judging from the fact that half of the molecules were degraded and the remaining half remained to be degraded, one can conclude that only one of the S enantiomer or the R enantiomer of the phosphorothioate derivative is a substrate of a degradation enzyme in serum.

### Example 9: A nucleic acid conjugate in which DNA strand bound to the 3' terminus of an RNA strand.

In the nucleic acid conjugate used in the previous experiments, the DNA was bound to the 5' terminus of the sense strand of the siRNA. In order to examine whether degradation takes place at the first RNA residue bound to the DNA even in cases where the DNA is bound to the 3' terminus of the sense strand of the siRNA, a nucleic acid conjugate in which the DNA portion and the siRNA portion were arranged in a manner to be opposite to that the nucleic acid conjugates used in previous experiments (a nucleic acid conjugate comprising a poly(dA) strand bound to the 3' terminus of an oligoribonucleotide represented by SEQ ID NO: 1) was provided and an experiment was performed.

The gel fluorescence image thus obtained is shown in FIG. 8. No change in molecular weight arising from incubation in serum was observed even in the nucleid acid complex in which the DNA was bound to the 3' terminus of the sense strand of the siRNA (FIG. 8, lanes 3 and 4). This result suggests that a degradation enzyme precisely recognizes a DNA in the direction from the 5' terminus to the 3' terminus, or alternatively a DNA extending from the 5' terminus of the double-stranded RNA strand to the 3' terminus of the DNA.

The results mentioned above indicate that, for site-specific degradation of a DNA-RNA nucleic acid conjugate in serum, the DNA portion may have any sequence and the presence of the double-stranded RNA portion is indispensable.

To avoid such degradation, substituting the hydroxyl group at the 2' position of the first RNA residue bound to a DNA strand (substitution at the second or following RNA residuebound to a DNA strand shows no significant effect) with an alkoxy group such as methoxy group or a halogen atom such as fluorine atom is required, or alternatively substituting the phosphate diester group between the 3' position of the first ribonucleotide bound to the 3' terminus of a DNA strand and the 5' position of the second ribonucleotide by any of phosphorothioate group, dithiophosphate diester group and trithiophosphate diester group is required.

### Example 10: Preparation of a complex of polynucleotide and schizophyllan.

### (1) Preparation of a triple-helix schizophyllan

A schizophyllan species having a triple helix structure was prepared according to conventional methods described in literatures (A. C. S. 38(1), 253(1997); Carbohydrate Research, 89, 121-135 (1981)). That is, *Schizophyllum commune* Fries (ATCC 44200) obtained from ATCC (American Type Culture Collection) was cultured using a minimal medium with shaking for 7 days and the supernatant obtained by centrifugal separation of cell components and insoluble was subjected to ultrasonic treatment, which was ultrafiltrated to replace the solution with water and lyophilized to obtain a triple-helix schizophyllan having a molecular weight of 450,000.

### (2) Preparation of a complex

The schizophyllan species obtained as described above was dissolved in a 0.25 N aqueous solution of NaOH to a concentration of 15 g/dL. To 10 µL of this solution, 10 µL of phosphate buffer (pH = 4.5) and 30 µL of a 3 g/dL solution of polynucleotide were mixed and to prepare an aqueous solution of a complex. The solution thus obtained was clear and homogeneous.

## Claims

1. A nucleic acid conjugate comprising a single-stranded DNA having nucleotides not less than 10 and a double-stranded RNA, wherein the 3' position of the 3'-terminal deoxyribonucleotide residue of the single-stranded DNA is bound to the 5' position of the 5'-terminal ribonucleotide residue of one of the ribonucleotide strands of the double-stranded RNA, and the hydroxyl group at the 2' position of the 5'-terminal nucleotide of the ribonucleotide strand, which is bound to the single-stranded DNA, is substituted with an alkoxy group or fluorine atom.

2. A nucleic acid conjugate comprising a single-stranded DNA having nucleotides not less than 10 and a double-stranded RNA, wherein the 3' position of the 3'-terminal deoxyribonucleotide residue of the single-stranded DNA is bound to the 5' position of the 5'-terminal ribonucleotide residue of one of the ribonucleotide strands of the double-stranded RNA, and the phosphate diester group between the 3' position of the first ribonucleotide bound to the single-stranded DNA and the 5' position of the adjacent ribonucleotide is substituted with any of phosphorothioate group, dithiophosphate diester group, and trithiophosphate diester group.

3. The nucleic acid conjugate according to any one of Claims 1 or 2, wherein the single-stranded DNA is a polydeoxyadenine strand.

4. The nucleic acid conjugate according to any one of Claims 1 to 3, wherein at least some of the phosphate diester groups of the single-stranded DNA are substituted with any of phosphorothioate group, dithiophosphate diester group and trithiophosphate diester group.

5. The nucleic acid conjugate according to Claim 4, wherein at least 50% or more of the phosphate diester groups of the single-stranded DNA are substituted with any of phosphorothioate group, dithiophosphate diester group and trithiophosphate diester group.

6. The nucleic acid conjugate according to any one of Claims 1 to 5, wherein the double-stranded RNA is an siRNA.

7. The nucleic acid conjugate according to Claim 6, wherein the siRNA is a 21-mer or 27-mer siRNA.

8. The nucleic acid conjugate according to Claim 6 or 7, wherein one or more target genes of the siRNA are genes expressed in Dectin-1-expressing cells.

9. A nucleic acid-polysaccharide complex, wherein the single-stranded DNA portion of one molecule of the nucleic acid conjugate according to any one of Claims 1 to 8 and two molecules of a polysaccharide having a β-1,3-glucan backbone form a triple helix structure.

10. The nucleic acid-polysaccharide complex according to Claim 9, wherein the polysaccharide having a β-1,3-glucan backbone is schizophyllan.

11. A pharmaceutical composition comprising the nucleic acid-polysaccharide complex according to Claim 10.

12. A method of enhancing the stability of a nucleic acid conjugate to a ribonuclease, wherein the nucleic acid conjugate comprises a single-stranded DNA having nucleotides not less than 10 and a double-stranded RNA; and the 3' position of the 3'-terminal deoxyribonucleotide residue of the single-stranded DNA is bound to the 5' position of the 5'-terminal ribonucleotide residue of one of the ribonucleotide strands of the double-stranded RNA; the method comprising substituting the hydroxyl group at the 2' position of the 5'-terminal nucleotide of the ribonucleotide strand, which is bound to the single-stranded DNA, by an alkoxy group or fluorine atom to stabilize the nucleic acid conjugate.

13. A method of enhancing the stability of a nucleic acid conjugate to a ribonuclease, wherein the nucleic acid conjugate comprises a single-stranded DNA having nucleotides not less than 10 and a double-stranded RNA; and the 3' position of the 3'-terminal deoxyribonucleotide residue of the single-stranded DNA is bound to the 5' position of the 5'-terminal ribonucleotide residue of one of the ribonucleotide strands of the double-stranded RNA; the method comprising substituting the phosphate diester group between the 3' position of the first ribonucleotide bound to the single-stranded DNA and the 5' position of the adjacent ribonucleotide by any of phosphorothioate group, dithiophosphate diester group and trithiophosphate diester group to stabilize the nucleic acid conjugate.

## Patentansprüche

1. Nukleinsäure-Konjugat, umfassend eine einzelsträngige DNA mit nicht weniger als 10 Nukleotiden und eine doppelsträngige RNA, wobei die 3'-Position des 3'-terminalen Desoxyribonukleotidrests der einzelsträngigen DNA an die 5'-Position des 5'-terminalen Ribonukleotidrests von einem der Ribonukleotidstränge der doppelsträngigen RNA gebunden ist und die Hydroxylgruppe an der 2'-Position des 5'-terminalen Nukleotids des Ribonukleotidstrangs, der an die einzelsträngige DNA gebunden ist, mit einer Alkoxygruppe oder einem Fluoratom substituiert ist.

2. Nukleinsäure-Konjugat, umfassend eine einzelsträngige DNA mit nicht weniger als 10 Nukleotiden und eine doppelsträngige RNA, wobei die 3'-Position des 3'-terminalen Desoxyribonukleotidrests der einzelsträngigen DNA an die 5'-Position des 5'-terminalen Ribonukleotidrests von einem der Ribonukleotidstränge der doppelsträngigen RNA gebunden ist und die Phosphatdiestergruppe zwischen der 3'-Position des ersten an die einzelsträngige DNA gebundenen Ribonukleotids und der 5'-Position des benachbarten Ribonukleotids mit einer aus Phosphorthioatgruppe, Dithiophosphatdiestergruppe und Trithiophosphatdiestergruppe substituiert ist.

3. Nukleinsäure-Konjugat nach einem von Anspruch 1 oder 2, wobei die einzelsträngige DNA ein Polydesoxyadenin-Strang ist.

4. Nukleinsäure-Konjugat nach einem der Ansprüche 1 bis 3, wobei mindestens einige der Phosphatdiestergruppen der einzelsträngigen DNA mit einer aus Phosphorthioatgruppe, Dithiophosphatdiestergruppe und Trithiophosphatdiestergruppe substituiert sind.

5. Nukleinsäure-Konjugat nach Anspruch 4, wobei mindestens 50% oder mehr der Phosphatdiestergruppen der einzelsträngigen DNA mit einer aus Phosphorthioatgruppe, Dithiophosphatdiestergruppe und Trithiophosphatdiestergruppe substituiert sind.

6. Nukleinsäure-Konjugat nach einem der Ansprüche 1 bis 5, wobei die doppelsträngige RNA eine siRNA ist.

7. Nukleinsäure-Konjugat nach Anspruch 6, wobei die siRNA eine 21-mer-oder 27-mer-siRNA ist.

8. Nukleinsäure-Konjugat nach Anspruch 6 oder 7, wobei ein oder mehrere Zielgene der siRNA Gene sind, die in Dectin-1-exprimierenden Zellen exprimiert werden.

9. Nukleinsäure-Polysaccharid-Komplex, wobei der einzelsträngige DNA-Abschnitt eines Moleküls des Nukleinsäure-Konjugats nach einem der Ansprüche 1 bis 8 und zwei Moleküle eines Polysaccharids mit einer β-1,3-Glucan-Rückgrat eine Dreifachhelixstruktur bilden.

10. Nukleinsäure-Polysaccharid-Komplex nach Anspruch 9, wobei das Polysaccharid mit einem β-1,3-Glucan-Rückgrat Schizophyllan ist.

11. Pharmazeutische Zusammensetzung, umfassend den Nukleinsäure-Polysaccharid-Komplex nach Anspruch 10.

12. Verfahren zur Verstärkung der Stabilität eines Nukleinsäure-Konjugats gegenüber einer Ribonuklease, wobei das Nukleinsäure-Konjugat eine einzelsträngige DNA mit nicht weniger als 10 Nukleotiden und eine doppelsträngige RNA umfasst; und die 3'-Position des 3'-terminalen Desoxyribonukleotidrests der einzelsträngigen DNA an die 5'-Position des 5'-terminalen Ribonukleotidrests von einem der Ribonukleotidstränge der doppelsträngigen RNA gebunden ist; wobei das Verfahren das Substituieren der Hydroxylgruppe an der 2'-Position des 5'-terminalen Nukleotids des Ribonukleotidstrangs, der an die einzelsträngige DNA gebunden ist, mit einer Alkoxygruppe oder einem Fluoratom umfasst, um das Nukleinsäure-Konjugat zu stabilisieren.

13. Verfahren zur Verstärkung der Stabilität eines Nukleinsäure-Konjugats gegenüber einer Ribonuklease, wobei das Nukleinsäure-Konjugat eine einzelsträngige DNA mit nicht weniger als 10 Nukleotiden und eine doppelsträngige RNA umfasst; und die 3'-Position des 3'-terminalen Desoxyribonukleotidrests der einzelsträngigen DNA an die 5'-Position des 5'-terminalen Ribonukleotidrests von einem der Ribonukleotidstränge der doppelsträngigen RNA gebunden ist; wobei das Verfahren das Substituieren der Phosphatdiestergruppe zwischen der 3'-Position des ersten an die einzelsträngige DNA gebundenen Ribonukleotids und der 5'-Position des benachbarten Ribonukleotids mit einer aus Phosphorthioatgruppe, Dithiophosphatdiestergruppe, und Trithiophosphatdiestergruppe umfasst, um das Nukleinsäure-Konjugat zu stabilisieren.

## Revendications

1. Conjugué d'acides nucléiques comprenant un ADN simple brin comportant pas moins de 10 nucléotides et un ARN double brin, dans lequel la position 3' du résidu de désoxyribonucléotide terminal en 3' de l'ADN simple brin est liée à la position 5' du résidu de ribonucléotide terminal en 5' de l'un des brins de ribonucléotides de l'ARN double brin, et le groupe hydroxyle en position 2' du nucléotide terminal en 5' du brin de ribonucléotides, qui est lié à l'ADN simple brin, est substitué par un groupe alcoxy ou un atome de fluor.

2. Conjugué d'acides nucléiques comprenant un ADN simple brin comportant pas moins de 10 nucléotides et un ARN double brin, dans lequel la position 3' du résidu de désoxyribonucléotide terminal en 3' de l'ADN simple brin est liée à la position 5' du résidu de ribonucléotide terminal en 5' de l'un des brins de ribonucléotides de l'ARN double brin, et le groupe diester de phosphate entre la position 3' du premier ribonucléotide lié à l'ADN simple brin et la position 5' du ribonucléotide adjacent est substitué par l'un quelconque d'un groupe phosphorothioate, d'un groupe diester de dithiophosphate, et d'un groupe diester de trithiophosphate.

3. Conjugué d'acides nucléiques selon l'une quelconque des revendications 1 ou 2, dans lequel l'ADN simple brin est un brin de polydésoxyadénine.

4. Conjugué d'acides nucléiques selon l'une quelconque des revendications 1 à 3, dans lequel au moins certains des groupes diester de phosphate de l'ADN simple brin sont substitués par l'un quelconque d'un groupe phosphorothioate, d'un groupe diester de dithiophosphate, et d'un groupe diester de trithiophosphate.

5. Conjugué d'acides nucléiques selon la revendication 4, dans lequel au moins 50 % ou plus des groupes diester de phosphate de l'ADN simple brin sont substitués par l'un quelconque d'un groupe phosphorothioate, d'un groupe diester de dithiophosphate, et d'un groupe diester de trithiophosphate.

6. Conjugué d'acides nucléiques selon l'une quelconque des revendications 1 à 5, dans lequel l'ARN double brin est un siARN.

7. Conjugué d'acides nucléiques selon la revendication 6, dans lequel le siARN est un siARN 21-mer ou 27-mer.

8. Conjugué d'acides nucléiques selon la revendication 6 ou 7, dans lequel un ou plusieurs gènes cibles du siARN sont des gènes exprimés dans des cellules exprimant la dectine 1.

9. Complexe acide nucléique-polysaccharide, dans lequel la partie ADN simple brin d'une molécule du conjugué d'acides nucléiques selon l'une quelconque des revendications 1 à 8 et deux molécules d'un polysaccharide comportant un squelette de β-1,3-glucane forment une structure de triple hélice.

10. Complexe acide nucléique-polysaccharide selon la revendication 9, dans lequel le polysaccharide comportant un squelette β-1,3-glucane est un schizophyllane.

11. Composition pharmaceutique comprenant le complexe acide nucléique-polysaccharide selon la revendication 10.

12. Procédé d'amplification de la stabilité d'un conjugué d'acides nucléiques envers une ribonucléase, dans lequel le conjugué d'acides nucléiques comprend un ADN simple brin comportant pas moins de 10 nucléotides et un ARN double brin ; et la position 3' du résidu de désoxyribonucléotide terminal en 3' de l'ADN simple brin est liée à la position 5' du résidu de ribonucléotide terminal en 5' de l'un des brins de ribonucléotides de l'ARN double brin ; le procédé comprenant la substitution du groupe hydroxyle en position 2' du nucléotide terminal en 5' du brin de ribonucléotides, qui est liée à l'ADN simple brin, par un groupe alcoxy ou un atome de fluor pour stabiliser le conjugué d'acides nucléiques.

13. Procédé d'amplification de la stabilité d'un conjugué d'acides nucléiques envers une ribonucléase, dans lequel le conjugué d'acides nucléiques comprend un ADN simple brin comportant des nucléotides pas moins de 10 et un ARN double brin ; et la position 3' du résidu de désoxyribonucléotide terminal en 3' de l'ADN simple brin est liée à la position 5' du résidu de ribonucléotide terminal en 5' de l'un des brins de ribonucléotides de l'ARN double brin ; le procédé comprenant la substitution du groupe diester de phosphate entre la position 3' du premier ribonucléotide lié à l'ADN simple brin et la position 5' du ribonucléotide adjacent par l'un quelconque d'un groupe phosphorothioate, d'un groupe diester de dithiophosphate, et d'un groupe diester de trithiophosphate pour stabiliser le conjugué d'acides nucléiques.


### SEQUENCE LISTING

<110> Japan Science and Technology Agency
<120> Composite nucleic acid and nucleic acid-polysaccharide complex
<130> 13F050-PCT
<150> JP 2012-139250
   <151> 2012-06-20
<160> 2
<170> PatentIn version 3.1
<210> 1
   <211> 21
   <212> RNA
   <213> Artificial Sequence
<220>
   <223> Sence sequence of siRNA
<400> 1
   caaagacaac caacuagugg u 21
<210> 2
   <211> 21
   <212> DNA
   <213> Artificial Sequence
<220>
   <223> Antisence sequence of siRNA
<400> 2
   accacuaguu gguugucuuu g 21